# EUROPEAN PATENT APPLICATION

(11) **EP 4 609 887 A1**
(43) Date of publication of application: **03.09.2025**
(21) Application number: 24159987.7
(22) Date of filing: 27.02.2024
(51) Int. Cl.: A61M 1/00, A61B 5/00

(54) **NEGATIVE PRESSURE WOUND THERAPY**

(71) Applicant: Mölnlycke Health Care AB, 402 52 Göteborg (SE)
(72) Inventor: BENGTSSON, Erik, 431 41 Mölndal (SE)
(74) Representative: Zacco Sweden AB

(57) **Abstract**

An apparatus for providing negative pressure to a wound site and related aspects are disclosed. The apparatus comprises a source of negative pressure configured to provide negative pressure via a fluid flow path from an inlet of the source of negative pressure to a wound site, a gas concentration sensor configured to detect concentration levels of one or more gases within the fluid flow path or at the wound site, and control circuitry operatively connected to the source of negative pressure and the gas concentration sensor. The control circuitry is configured to output a blockage signal indicative of a blockage being present within the fluid flow path based on a detected concentration level of one or more gases within the fluid flow path over time, and/or output a leakage signal indicative of a leakage being present within the fluid flow path based on a detected concentration level of one or more gases within the fluid flow path over time.

## Description

### TECHNICAL FIELD

The herein disclosed embodiments generally relate to negative pressure wound therapy (NPWT). In particular, the herein disclosed embodiments relate to apparatuses for providing reduced pressure to a wound site, methods for controlling an apparatus for providing reduced pressure to a wound site, and thereto related computer program products and computer-readable storage media.

### BACKGROUND

Negative pressure wound therapy (NPWT) is a technique that promotes healing of e.g. surgical, acute and chronic wounds by the application of a sub-atmospheric pressure ("negative pressure") to the wound site, using a negative pressure pump. Wound healing is achieved by applying a negative pressure, such as "vacuum" through a dressing or a cover applied onto the wound. Excess wound exudate is thereby drawn out, which increases the blood flow to the area, and promotes the formation of granulation tissue. The NPWT technique also permits less outside disturbance of the wound and transports excess fluids away from the wound site.

The NPWT technique has, historically, mainly been applied to a patient while in a hospital environment. However, recent product development allows the technique to be used by a patient in a home environment.

In a hospital setting, the wound to be treated is typically an open cavity wound, which is first filled with a wound filler, such as a gauze or a foam. The wound may thereafter be sealed with an adhesive film, and connected to a negative pressure pump via a drain or a port. The size of the foam, gauze and/or the adhesive film may be adapted and cut depending on the size, shape or type of wound. The application procedure is typically carried out by a caregiver. The negative pressure pump used in such a system is typically of a large size and generally has a high capacity to be able to deal with large amounts of wound exudate.

In a home environment, a portable NPWT device, which may be carried around by the patient, is generally preferred. A portable NPWT device typically comprises an absorbent dressing configured to be connected to a negative pressure source by means of a tubing. The pump used is in such devices is typically of a smaller size, and has a more limited capacity. An NPWT system comprising an absorbent dressing is also utilized in a hospital or care facility, particularly on less exuding or "closed" wounds, such as surgically closed incisions.

In some of the portable NPWT systems, the dressing serves as the sole means to collect wound exudate, whereas in other portable NPWT systems and in the NPWT systems adapted for the treatment of wounds in a hospital setting, a fluid collection means, such as a canister, arranged remote from the wound site, is included. In such systems, the canister serves as the predominant means for collection of wound exudate.

Regardless of whether the NPWT system comprises a non-absorbent or an absorbent dressing or whether the application procedure is to be carried out by a caregiver or a wearer in his/her home environment, there is room for improvements in this field.

For example, negative pressure applied to the wound site may need to be properly managed to maintain or increase the effectiveness of the negative pressure treatment. Moreover, leaks and blockages in some of the components of the NPWT apparatus may need to be detected and corrected to maintain effective treatment. For example, a leak or blockage in the tube that connects the negative pressure source to the dressing covering the wound site may disrupt the reduced pressure treatment being administered to the wound site.

### SUMMARY

The herein disclosed technology seeks to mitigate, alleviate or eliminate one or more of the above-identified deficiencies and disadvantages in the prior art to address various problems relating to blockage detection or leakage detection in an NPWT system.

Various aspects and embodiments of the disclosed technology are defined below and in the accompanying independent and dependent claims.

An aspect of the disclosed technology comprises an apparatus for providing negative pressure to a wound site. The apparatus comprises a source of negative pressure configured to provide negative pressure via a fluid flow path from an inlet of the source of negative pressure to a wound site, a gas concentration sensor configured to detect concentration levels of one or more gases within the fluid flow path or at the wound site, and control circuitry operatively connected to the source of negative pressure and the gas concentration sensor. The control circuitry is configured to output a blockage signal indicative of a blockage being present within the fluid flow path based on a detected concentration level of one or more gases within the fluid flow path over time, and/or output a leakage signal indicative of a leakage being present within the fluid flow path based on a detected concentration level of one or more gases within the fluid flow path over time.

Another aspect of the disclosed technology comprises a computer-implemented method for operating an apparatus for providing negative pressure to a wound site. The apparatus comprises a source of negative pressure configured to provide negative pressure via a fluid flow path from an inlet of the source of negative pressure to a wound site, and a gas concentration sensor configured to detect concentration levels of one or more gases within the fluid flow path or at the wound site. The method comprises outputting a blockage signal indicative of a blockage being present within the fluid flow path based on a detected concentration level of one or more gases within the fluid flow path over time, and/or outputting a leakage signal indicative of a leakage being present within the fluid flow path based on a detected concentration level of one or more gases within the fluid flow path over time.

Another aspect of the disclosed technology comprises a computer program product comprising instructions which, when the program is executed by a computing device of an apparatus for providing negative pressure to a wound dressing, causes the apparatus to carry out the method according to any one of the embodiments disclosed herein. With this aspect of the disclosed technology, similar advantages and preferred features are present as in the other aspects.

Another aspect of the disclosed technology comprises a (non-transitory) computer-readable storage medium comprising instructions which, when executed by a computing device of an apparatus for providing negative pressure to a wound dressing, causes the apparatus to carry out the method according to any one of the embodiments disclosed herein. With this aspect of the disclosed technology, similar advantages and preferred features are present as in the other aspects.

The term "non-transitory," as used herein, is intended to describe a computer-readable storage medium (or "memory") excluding propagating electromagnetic signals, but are not intended to otherwise limit the type of physical computer-readable storage device that is encompassed by the phrase computer-readable medium or memory. For instance, the terms "non-transitory computer readable medium" or "tangible memory" are intended to encompass types of storage devices that do not necessarily store information permanently, including for example, random access memory (RAM). Program instructions and data stored on a tangible computer-accessible storage medium in non-transitory form may further be transmitted by transmission media or signals such as electrical, electromagnetic, or digital signals, which may be conveyed via a communication medium such as a network and/or a wireless link. Thus, the term "non-transitory", as used herein, is a limitation of the medium itself (i.e., tangible, not a signal) as opposed to a limitation on data storage persistency (e.g., RAM vs. ROM).

Another aspect of the disclosed technology comprises a wound treatment system comprising apparatus according to any one of the embodiments disclosed herein, a wound cover for creating a sealed space defined in part by the wound site, and a tubing assembly defining the fluid flow path.

The disclosed aspects and preferred embodiments may be suitably combined with each other in any manner apparent to anyone of ordinary skill in the art, such that one or more features or embodiments disclosed in relation to one aspect may also be considered to be disclosed in relation to another aspect or embodiment of another aspect.

An advantage of some embodiments is that blockage conditions and/or leakage conditions may be detected independently from pressure readings.

An advantage of some embodiments is that additional blockage detection and leakage detection functionality is added to the apparatus, thereby increasing robustness and overall patient safety and comfort.

An advantage of some embodiments is that a health status of the wound can be automatically detected by the apparatus and appropriate measures can be taken, thereby increasing overall patient safety and comfort.

An advantage of some embodiments is that automatic monitoring of the amount of liquids being exuded from the wound is enabled, thereby allowing for a better adaptation of the durations of the pressure regulation cycles of the apparatus and accordingly reduced power consumption and/or improved patient safety and comfort.

Further embodiments are defined in the dependent claims. It should be emphasized that the term "comprises/comprising" when used in this specification is taken to specify the presence of stated features, integers, steps, or components. It does not preclude the presence or addition of one or more other features, integers, steps, components, or groups thereof.

These and other features and advantages of the disclosed technology will in the following be further clarified with reference to the embodiments described hereinafter.

### BRIEF DESCRIPTION OF THE DRAWINGS

The above aspects, features and advantages of the disclosed technology, will be more fully appreciated by reference to the following illustrative and non-limiting detailed description of example embodiments of the present disclosure, when taken in conjunction with the accompanying drawings, in which:
Fig. 1 is a schematic illustration of a wound treatment system in accordance with some embodiments.
Fig. 2 is a schematic illustration of a wound treatment system in accordance with some embodiments.
Fig. 3 is a schematic flowchart representation of a method for operating an apparatus for providing negative pressure to a wound site in accordance with some embodiments.

### DETAILED DESCRIPTION

The present disclosure will now be described in detail with reference to the accompanying drawings, in which some example embodiments of the disclosed technology are shown. The disclosed technology may, however, be embodied in other forms and should not be construed as limited to the disclosed example embodiments. The disclosed example embodiments are provided to fully convey the scope of the disclosed technology to the skilled person. Like reference characters refer to like elements throughout. Those skilled in the art will appreciate that the steps, services and functions explained herein may be implemented using individual hardware circuitry, using software functioning in conjunction with hardware circuitry such as a programmed microprocessor or general-purpose computer, using one or more Application Specific Integrated Circuits (ASICs), using one or more Field Programmable Gate Arrays (FPGA) and/or using one or more Digital Signal Processors (DSPs).

It will also be appreciated that when the present disclosure is described in terms of a method, it may also be embodied in an apparatus comprising one or more processors, one or more memories coupled to the one or more processors, where computer code is loaded to implement the method. For example, the one or more memories may store one or more computer programs that cause the apparatus to perform the steps, services and functions disclosed herein when executed by the one or more processors in some embodiments.

It is also to be understood that the terminology used herein is for purpose of describing particular embodiments only, and is not intended to be limiting. It should be noted that, as used in the specification and the appended claim, the articles "a", "an", "the", and "said" are intended to mean that there are one or more of the elements unless the context clearly dictates otherwise. Thus, for example, reference to "a unit" or "the unit" may refer to more than one unit in some contexts, and the like. Furthermore, the words "comprising", "including", "containing" do not exclude other elements or steps. It should be emphasized that the term "comprises/comprising" when used in this specification is taken to specify the presence of stated features, integers, steps, or components. It does not preclude the presence or addition of one or more other features, integers, steps, components, or groups thereof. The term "and/or" is to be interpreted as meaning "both" as well and each as an alternative. Similarly, "at least one of A and B" is to be interpreted as only A, only B, or both A and B.

It will also be understood that, although the term first, second, etc. may be used herein to describe various elements or features, these elements should not be limited by these terms. These terms are only used to distinguish one element from another. For example, a first signal could be termed a second signal, and, similarly, a second signal could be termed a first signal, without departing from the scope of the embodiments. The first signal and the second signal are both signals, but they are not the same signal. The term "operatively connected" is in the context of the present disclosure to be understood as that the "operatively connected" entities or units can transmit and/or receive signals to and/or from each other.

As used herein, the term "in response to" may be construed to mean "when or "upon" or "if" depending on the context. Similarly, the phrase "if it is determined' or "when it is determined" or "in an instance of" may be construed to mean "upon determining or "in response to determining" or "upon detecting and identifying occurrence of an event" or "in response to detecting occurrence of an event" depending on the context. Accordingly, the phrase "if X equals Y" may be construed as "when X equals Y", "when it is determined that X equals Y", "in response to X being equal to Y", or "in response to detecting/determining that X equals Y" depending on the context.

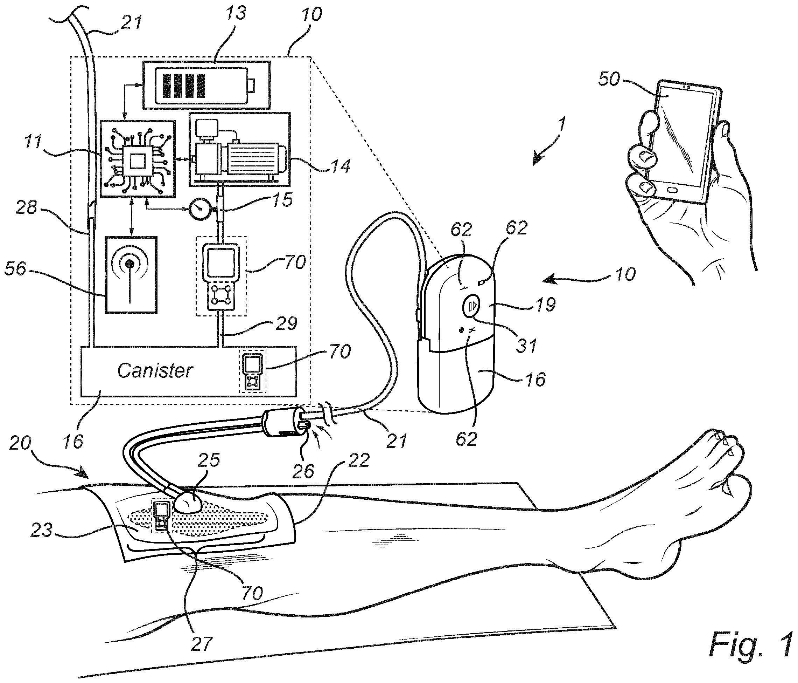

Turning now to the drawings, and to Fig. 1 in particular, there is schematically illustrated a wound treatment system 1 (sometimes simply referred to as "the system" 1) in accordance with some embodiments. The depicted wound treatment system 1 may be referred to as an "NPWT system" 1, "reduced pressure wound treatment system" 1, or "negative pressure wound treatment system" 1. The wound treatment system 1 comprises an apparatus 10 for providing reduced pressure to a tissue site 27 (or otherwise referred to as a wound site 27).

The wound treatment system 1 further comprises a wound cover 22 that is adapted to create a sealed space 23 defined in part by a wound surface, such as at the skin of a user/person, at or around a wound of the user/person. Further, the apparatus 10 (may also be referred to as an NPWT device 10) is fluidly connected to the wound cover 22 using e.g. a tubing 21. The tubing 21 may be of any suitable flexible tubing fabricated from elastomeric or polymeric materials. The tubing 21 may connect to the wound dressing 20, or more specifically to the wound cover 22 of the wound dressing 20, via a suitable connector 25. The connector 25 may be adhered or otherwise attached to the wound cover 22. In particular, the connector 25 may be attached around an opening (not shown) formed in the wound cover 22. The tubing 21 may comprise one or two individual tubes.

The expression "wound cover" as used herein should be interpreted broadly as any wound site member, e.g. it can be a film sealed around a periphery of a wound site 27, where a wound filler may be used to fill the wound volume prior to the application of such wound cover. Wound cover may also refer to a backing layer of a wound dressing 20 comprising an additional layer(s) such as for example an absorbent layer and/or a spacer layer.

The apparatus 10 comprises a source of negative pressure 14 configured to provide negative pressure (i.e. sub-atmospheric pressure) via a fluid flow path from an inlet of the source of negative pressure 14 to the wound dressing 20, or more specifically to provide negative pressure under a wound cover. The fluid flow path ("exudate lumen") is at least partly defined by the tubing 21. The source of negative pressure 14 is indicated as "VP" ("Vacuum Pump") in the figure. In some embodiments, the source of negative pressure 14 comprises a negative pressure pump that together with a motor is adapted for establishing a negative pressure when the source of negative pressure 14 is operating, i.e. when it is in an active state or simply "active". The source of negative pressure may comprise any type of pump and motor that are biocompatible or otherwise suitable for use in an NPWT setting and capable of maintaining or drawing adequate and therapeutic vacuum levels (i.e., negative pressure levels). Preferably, the negative pressure level to be achieved is in a range between about -20 mmHg and about -300 mmHg. In some embodiments, a negative pressure range between about -80 mmHg and about - 140 mmHg is used. In some embodiments, a negative pressure range between about -115 mmHg and about -135 mmHg is used. In some embodiments, the negative pressure pump is a diaphragm pump, a peristaltic pump, piezoelectric pump or the like, in which a motor causes the moving parts to draw fluid from the wound site 27.

In the context of the present disclosure, it should be understood that the expressions "negative pressure", "sub-atmospheric pressure", "reduced pressure", or even "vacuum", as used interchangeably herein, generally refer to a pressure less than a local ambient pressure, such as the ambient pressure in a local environment external to a sealed therapeutic environment provided by a wound cover 22. In many cases, the local ambient pressure may also be the atmospheric pressure at which a patient is located. Thus, the "negative pressure" may be understood as a pressure difference between the ambient pressure and the pressure under the wound cover, where ambient pressure is generally set as 0 mmHg. Unless otherwise indicated, values of pressure stated herein are gauge pressures. Similarly, references to increases in negative pressure typically refer to a decrease in pressure, while decreases in negative pressure typically refer to an increase in pressure.

Further, in some embodiments the apparatus 10 comprises a canister 16 fluidly connected to the negative pressure source 14. The canister 16 may be formed from e.g. moulded plastic or the like, and possibly be a detachable component of the apparatus 10. Moreover, the canister 16 may further be at least partly transparent/translucent to permit viewing into the interior of the canister 16 to assist the user in determining the remaining capacity of the canister 16. As used herein, the term "fluidly connected" should be interpreted broadly and may comprise e.g. any form of tubing, conduits, or channels providing a fluid connection/communication between two components such as between the canister 16 and the negative pressure source 14 or the canister 16 and the wound dressing 20.

In some embodiments, the canister 16 comprises an inlet port 28 for allowing connection to the tubing 21. The inlet port 28 may also be formed elsewhere at the apparatus 10, however still fluidly connected to the canister 16. The connection between the inlet port 28 and the tubing 21 is a sealed connection, thus ensuring that no leakage is formed at the inlet port 28 during normal operation of the apparatus 10. The tubing 21 is preferably detachably connected to the inlet port 28 through conventional means including a friction fit, bayonet coupling, snap fit, barbed connector, or the like. The inlet port 28 may be moulded/formed from the same material and/or at the same time as forming the canister 16. A similar sealed connection (e.g. using a flange insulation/"O-ring") is formed between the canister 16 (at the outlet port 29) and the source of negative pressure 14. The canister 16 may form a part of the first fluid flow path.

In some embodiments, the apparatus comprises a housing 19 enclosing the negative pressure source 14. The canister 16 may be detachably connected to a housing 19 comprising the negative pressure source 14, whereby e.g. a full canister may be removed and replaced with an empty (new) canister. In such embodiments it may be desirable to provide e.g. the canister 16 and the housing 19 with some form of engagement means for securing the canister 16 to the housing such that the canister 16 is not unintentionally removed from the housing 19. The engagement means may in one embodiment comprise a pair of flexible protrusions extending from the canister 16 and adapted to engage with e.g. corresponding locking grooves provided at the housing 19.

However, in some embodiments, the wound treatment system 1 is a canister-less wound treatment system (not shown), where the wound exudate is collected in an absorbent layer, or the like, of the wound dressing 20. In such embodiments, the fluid flow path from the wound dressing 20 to the negative pressure source 14 is provided with one or more suitable filters (not shown) that are adapted to allow gas flow from the wound site 27 to the source of negative pressure 14 and block the flow of liquids from the wound site 27 to the source of negative pressure 14, as readily understood by the person skilled in the art.

In some embodiments, the apparatus 10 comprises a power source, such as e.g. a battery 13 for powering the apparatus 10. The battery 13 may preferably be of the rechargeable type but may alternatively be arranged to be disposable and thus to be changed once discharged. A specifically adapted battery pack may be used in relation to some embodiments. The apparatus 10 may be of single-use type (allowing use during one single treatment time duration) or multiple-use type (allowing use during several different treatment sessions).

The apparatus 10 further comprises control circuitry 11 (may also be referred to as "a control unit", "a controller", or the like) electrically connected to the battery 13 and the source of negative pressure 14. The control circuitry 11 is configured to control operation of the source of negative pressure 14. The control circuitry 11 may comprise a microprocessor, microcontroller, programmable digital signal processor or another programmable device. The control circuitry 11 may also, or instead, include an application specific integrated circuit (ASIC), a programmable gate array or programmable array logic, a programmable logic device, or a digital signal processor. Where the control circuitry 11 includes a programmable device such as the microprocessor, microcontroller or programmable digital signal processor mentioned above, the control circuitry 11 may further include computer executable code that controls operation of the programmable device. Thus, in some embodiments, the control circuitry 11 comprises one or more processors and a memory, where the memory contains instructions executable by the processor whereby the apparatus 10 is operative to execute any one of the method steps or functions disclosed herein.

Furthermore, in some embodiments, the apparatus 10 comprises one or more pressure sensors 15 arranged in fluid connection with the inlet of the source of negative pressure 14, the canister 16, and/or the sealed space 23. In the depicted embodiment of Fig. 1, the apparatus 10 comprises a pressure sensor 15 arranged in a fluid flow path between the canister 16 and the source of negative pressure 14. However, as readily understood by a person skilled in the art, the pressure sensor 15 may be located at any other suitable location to sense or detect a pressure within the fluid flow path between the source of negative pressure 14 and the wound site. For example, the pressure sensor 15 may be arranged within the canister or under the wound cover 22.

During use of the apparatus 10, the wound cover 22 is arranged at a wound site 27 of the user/patient, forming the sealed space 23. A tubing assembly is provided with a first tubing 21 to fluidly connect the wound cover 22 to the inlet port 28 of the apparatus 10. The apparatus 10 is then activated, e.g. by a user pressing a start/pause button 31 of a user-interface of the apparatus 10 that activates the source of negative pressure 14. Alternatively, activation may be initiated via an external device 50 connected to the system 1. The external device 50 is further described below. When activated, the source of negative pressure 14 will start to evacuate air through the canister 16, the inlet port 28, the tubing 21 and the sealed space 23 formed by the wound cover 22. Accordingly, a negative pressure will be created within the sealed space 23. This initial evacuation of air following a start-up of the apparatus 10 may be referred to as "depressurization". The initial evacuation of air (i.e., "depressurization") may continue until reaching a set pressure value (e.g., -125 mmHg). In some embodiments, the control circuitry 11 is configured to control the source of negative pressure 14 so to establish and maintain a negative pressure level (as measured by the pressure sensor 15) within a negative pressure range. The negative pressure range may for example be -20 mmHg to -300 mmHg, -80 to -180 mmHg, -100 to -150 mmHg, -110 to - 140 mmHg, -115 to -135 mmHg, or any suitable subrange thereof (e.g., -80 mmHg to -100 mmHg). The negative pressure range may for example be selected in view of the type of wound and/or the patient.

In case a liquid has been formed at the wound site 27, this liquid from the wound site 27 may at least partly be "drawn" from the wound site, through the tubing 21, the inlet port 28 and into the canister 16. The amount of liquid (possibly defined as exudate) that is drawn from the wound and collected in the canister 16 will depend on the type of wound that is being treated, the duration of the treatment, as well as the type of wound dressing 20 used. For example, in case an absorbent dressing is used, the liquid may be absorbed and collected both in the canister 16 and the wound dressing 20, whereas if a dressing with no absorption capacity or little absorption capacity is used, most or all of the liquid from the wound site 27 may be collected in the canister 16. A suitable filter member (not shown) is generally arranged in proximity to the outlet port 29 of the canister 16 to ensure that no liquid is allowed to pass to the source of negative pressure 14 from the canister 16.

In some embodiments, the wound treatment system 1 further comprise an air inlet port 26 or a controlled leakage port 26 that provides a small and controlled inflow of air (as indicated by the arrows) under the wound cover 22. The air inlet port 26 may for example be provided via a secondary lumen extending from the connector 25 as schematically illustrated. However, in some embodiments the air inlet port 26 may additionally or alternatively be provided at the connector 25 and/or at any suitable location of the tubing 21.

Accordingly, the wound treatment system 1 may in some embodiments be arranged to ensure that a small controlled leakage is present, ensuring that a flow from the sealed space 23 may be achieved (under the assumption that there is no general blocking e.g. within the tubing 21). Additionally, during use of the wound treatment system 1 some leakage may be expected in relation to the wound cover 22. In some embodiments, the air inlet port 26 includes means for supplying/providing air to the wound site 27 at a predetermined supply rate, the rate being from 2 to 7 ml/min, preferably from 3 to 5 ml/min at a predetermined level of negative pressure. The predetermined level of negative pressure may be a value within the range from -80 to -180 mmHg, preferably from -100 to -150 mmHg, more preferably from -110 to -140 mmHg, from -115 to -135 mmHg, or -120 to -135 mmHg. The means for providing air to the wound site 27 (i.e. under the wound cover 22) at a predetermined supply rate may for example be in the form of an air filter (not shown).

In some embodiments, the air filter (associated with the controlled leakage) comprises a hydrophobic and porous material, where the size of the pores is within the range of from 2 to 20 µm, preferably in the range of from 5 to 12 µm. The pore size of the filter is measured in a non-compressed state. In some embodiments, the air filter comprises polyethylene or sintered polyethylene.

Furthermore, the apparatus 10 may comprise a communication interface 56 having suitable components (e.g. transceivers, antennas, filters, power amplifiers, etc.) with suitable communication protocols (e.g., Wi-Fi, Bluetooth, Bluetooth Low Energy, Zigbee, or the like) in order to establish a connection with an external device 50 and to transmit and receive wireless signals to and from the external device 50. The communication interface 56, indicated as "Cl" is connected to the control circuitry 11, indicated as "CTRL". The communication interface 56 may be configured to transmit and receive wireless signals to and from an external handheld device 50. In more detail, the communication interface 56 comprises one or more transceivers and one or more antennas connected to the one or more transceivers. The one or more transceivers are accordingly configured to transmit signals to an external device 50 via the one or more antennas and to receive signals from an external device 50 via the one or more antennas. For example, the communications interface 56 may include a Wi-Fi transceiver for communicating via a wireless communications network or cellular or mobile phone communications transceivers. In another example, the communication interface 56 may include a short-range wireless transmitter (e.g., a Bluetooth wireless transmitter, etc.) for communicating via a short-range wireless communication transceiver.

The external device may be an external handheld device 50, such as a mobile phone (e.g. a smart phone) operatively connected to the apparatus 10. The term "operatively connected" is in the context of the present disclosure to be understood as that the "operatively connected" entities or units can transmit and/or receive signals to and/or from each other.

Still further, the apparatus 10 may comprise one or more Light Emitting Diodes (LEDs) 62. The one or more LEDs 62 may be considered to form a User Interface (Ul) together with the button 31 arranged on the housing 19. The LEDs 62 may for example be arranged on the housing 19 of the apparatus 10. The LEDs 62 may be arranged under partly transparent portions of the housing to indicate various operating conditions of the apparatus 10. In more detail, the LEDs may be used to indicate a State of Charge (SoC) or available capacity of the power source 13, presence of a blockage condition, and/or a presence of a leakage condition. The aforementioned transparent portions may accordingly be formed as suitable elements/icons indicating the various operating conditions of the apparatus 10 (e.g., a battery-shaped element/icon for indicating battery capacity). Moreover, each LED may be arranged to emit light of different colours (e.g., green, yellow, red) to provide more granularity in the indications. For example, a green battery icon may indicate high battery capacity (e.g., SoC > 50%), a yellow battery icon may indicate a moderate battery capacity (e.g., 50% > SoC > 10%), while a red battery icon may indicate a low battery capacity (e.g., SoC < 10%). The apparatus 10 may comprise further LEDs than those indicated in the drawings. For example, the apparatus 10 may comprise an LED associated with the button 31 in order to indicate an active state of the apparatus 10.

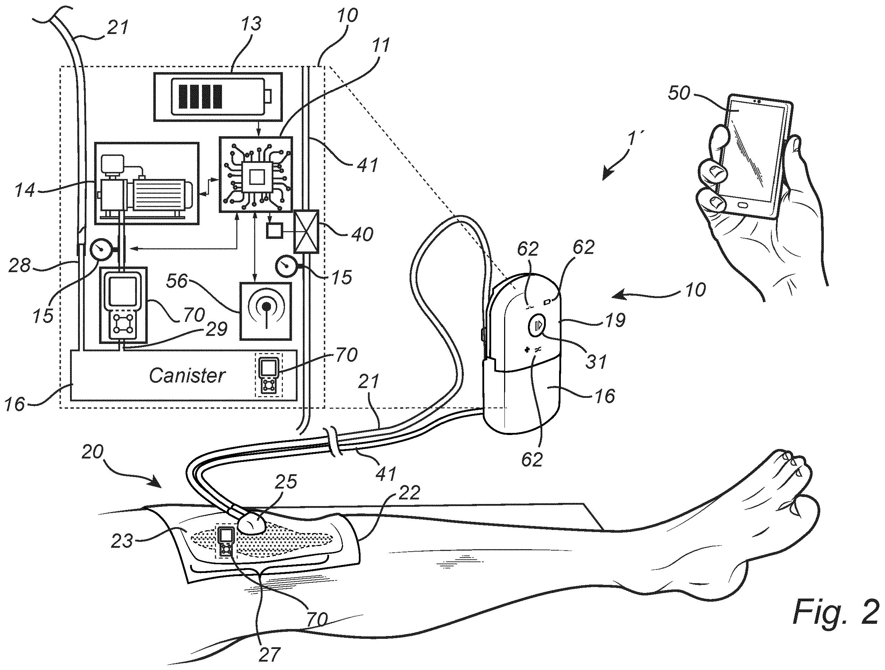

Fig. 2 depicts a wound treatment system 1' in accordance with some embodiments. Many functions, features and components of the wound treatment system 1' depicted in Fig. 2 are the same as or analogous to the wound treatment system 1 depicted in Fig. 1 and will for the sake of brevity and conciseness not be repeated. Instead, the focus will be on the differentiating functions, features, or components between the two wound treatment systems. Thus, as the person skilled in the art readily understands, unless specifically indicated, the foregoing discussion related to the components of the wound treatment system 1 with reference to Fig. 1 is applicable for the corresponding components depicted in Fig. 2 as indicated by the reference numerals.

In general, the wound treatment system 1 depicted in Fig. 1 differs from the wound treatment system 1' depicted in Fig. 2 in that the former is often referred to as a "single-lumen" NPWT system 1 while the latter is often referred to as a "dual-lumen" NPWT system 1'. Accordingly, while the wound treatment system 1 depicted in Fig. 1 has a "controlled inflow of air" via leakage (also referred to as a controlled leakage), the wound treatment system 1' depicted in Fig. 2 does not have a controlled leakage, but instead explicitly controls or regulates the air/fluid supply to the wound site 27 by means of the apparatus 10.

Therefore, in contrast to the wound treatment system 1 depicted in Fig. 1, the wound treatment system 1' of Fig. 2 comprises a second fluid flow path that fluidly connects the sealed space 23 created by the wound cover 22 to the ambient atmosphere or a "fluid reservoir" (not shown) via an electronically controllable valve 40. The second fluid flow path (may also be referred to as "air lumen") is at least partly defined by a second tubing 41 that may be of any suitable flexible tubing fabricated from elastomeric or polymeric materials. Here, the fluid flow path from the inlet of the source of negative pressure 14 to the wound dressing 20 may be construed as a "first" fluid flow path or "exudate lumen".

Moreover, the control circuitry 11 is electronically connected to the electronically controllable valve 40, and the control circuitry 11 is further configured to control an operation of the electronically controllable vale 40 so to release negative pressure from the wound site via the second fluid flow path (or "air lumen"). In other words, the control circuitry 11 is configured to open the electronically controllable valve 40 so to introduce fluid (e.g. air) to the wound site 27 when there is a sub-atmospheric pressure under the wound cover 22. This operation may also be referred to as "flushing".

Since the wound treatment system 1' depicted in Fig. 2 does not have any "controlled leakage flow", there is a risk that the apparatus 10 would not be able to draw any, or at least a sub-optimal amount of "exudate" from the wound site 27 to the canister 16 once negative pressure has been established under the wound cover 22 due to lack of airflow through the system 1'. Therefore, in order to be able to draw desired amounts of wound exudate from the wound site, a fluid, such as e.g. air from the ambient atmosphere, may be controllably introduced at defined time intervals and/or in response to pressure measurements by opening the electronically steerable valve 40. Once enough fluid has been introduced (e.g. in response to a negative pressure within the first and/or second fluid flow path reaching a lower threshold), the control circuitry 11 is configured to close the electronically steerable valve 40 and to activate the source of negative pressure 14.

The ability to controllably "flush" the system 1' by injecting air via an electronically steerable valve 40 may provide the advantage of longer pressure regulation cycles, which reduces the on-time for the source of negative pressure 14 and therefore reduces the energy consumption of the apparatus 10.

As before, the apparatus 10 of the wound treatment system 1' may comprise one or more pressure sensors 15 arranged in fluid connection with the inlet of the source of negative pressure 14, the canister 16, and/or the sealed space 23. However, the apparatus 10 may comprise a pressure sensor arranged in fluid connection with the second fluid flow path (i.e., "air lumen"). The pressure sensor 15 (i.e., "air lumen pressure sensor") is preferably arranged to measure a pressure level in the second fluid flow path downstream of the electronically controllable value 40 (i.e., between the valve 40 and the wound site 27). In the depicted embodiment of Fig. 2, the apparatus comprises two pressure sensors, a first pressure sensor configured to measure a pressure level within the first fluid flow path, and a second pressure sensor configured to measure a pressure level within the second fluid flow path. However, the apparatus 10 may comprise a single pressure sensor 15 arranged in any one of the suitable locations mentioned in the foregoing.

The apparatus 10 may further comprise an air filter (not shown) arranged in the second fluid flow path in order to limit the airflow when the electronically controllable valve 40 is open. In some embodiments, the air filter comprises a hydrophobic and porous material, where the size of the pores is configured to allow for a flow in the range of 50-120 ml/s, such as 60-100 ml/s when the valve 40 is opened after negative pressure has been established under the wound cover 20. The pore size of the filter is preferably measured in a non-compressed state. In some embodiments, the air filter comprises polyethylene or sintered polyethylene. The air filter may also serve to reduce the risk of contaminants entering the wound site 27 when the valve 40 is opened (i.e., during "flushing"). The air filter may be arranged in the tube 41 that at least partly defines the second fluid flow path. The air filter may be arranged upstream of the valve 40 (i.e., between the valve 40 and the inlet of ambient air, or downstream of the valve 40 such as between the valve and the air lumen pressure sensor 15.

Furthermore, regardless of the wound treatment system 1, 1' being single lumen (Fig. 1) or dual lumen (Fig. 2), the wound treatment system 1, 1' comprises a gas concentration sensor 70 configured to detect concentration levels of one or more gases within the fluid flow path or at the wound site 27. The gas concentration sensor may be arranged in a fluid flow path between the canister 16 and the source of negative pressure 14, in the canister 16, or in the wound dressing 20, as indicated in Fig. 1 and Fig. 2. The apparatus may comprise more than one gas concentration sensor 70 arranged in two or more of the aforementioned locations. The gas concentration sensor may be of any suitable sensor type such as e.g., electrochemical sensors, metal oxide semiconductor (MOS) sensors, infrared sensors or other optical sensors. In some embodiments, the gas concentration sensor is a Non-Dispersive Infrared (NDIR) sensor.

Non-dispersive infrared (NDIR) sensors are devices used for detecting and measuring the concentration of gases in the environment. They work based on the principle that molecules of certain gases absorb infrared light at specific wavelengths. In more detail, NDIR sensors have an infrared light source that emits infrared light across a broad spectrum, including wavelengths specific to the gas being measured. The emitted infrared light then passes through the gas sample, and if the gas of interest is present in the sample, molecules of that gas absorb specific wavelengths of the infrared light. After passing through the gas sample, the remaining infrared light is detected by a detector that is sensitive to the wavelengths of light that were not absorbed by the gas. Thus, by comparing the intensity of the infrared light before and after it passes through the gas sample, the sensor can determine the concentration of the gas based on the amount of absorption that occurred. The sensor may be configured to process the detected signal to calculate the gas concentration and then output it to a control system (e.g., the control circuitry 11).

In some embodiments, the gas concentration sensor 70 is configured to detect concentration levels of gases such as, oxygen (O₂), methane (CH₄), carbon dioxide (CO₂), ammonia (NH₃), Hydrogen Sulfide (H₂S), and volatile organic compounds (VOCs). The concentration of Hydrogen Sulfide, Ammonia, Methane, and VOCs, such as e.g., alcohols, aldehydes, and ketones, may be used as indicators of a health status of a wound (e.g., presence of infections). In particular, the gas concentration sensor may be configured to detect concentration levels of carbon dioxide (CO₂). Preferably, the gas concentration sensor 70 is configured to detect concentration levels of one or more atmospheric gases.

Furthermore, the control circuitry 11 is operatively connected to the gas concentration sensor 70 and configured to output a blockage signal indicative of a blockage being present within the fluid flow path based on a detected concentration level of one or more gases within the fluid flow path over time. In particular, the control circuitry 11 may be configured to output the blockage signal indicative of a blockage being present within the fluid flow path based on a detected concentration level of one or more gases within the fluid flow path over time while the source of negative pressure 14 is active. In some embodiments, the control circuitry 11 may be configured to output the blockage signal indicative of a blockage being present within the fluid flow path in response to a detected concentration level of the one or more gases within the fluid flow path being non-responsive or non-changing while the source of negative pressure 14 is active. Here, non-responsive or non-changing concentration level does not necessarily mean that the concentration level has to be perfectly static, but that it is substantially non-responsive (i.e., static within some suitable margin of error). The margin of error may for example be defined based on the specifications of the applied gas concentration sensor 70.

In other words, if the source if negative pressure 14 is active, the expected response for the gas concentration sensor would be that the concentration level of one or more gases (e.g., CO₂) will decrease. However, if the concentration level is not decreasing, this is treated as an indicator of a blockage being present in the fluid flow path, and in particular, a blockage downstream of the gas concentration sensor, (i.e., between the gas concentration sensor and the source of negative pressure). Therefore, in some embodiments, the gas concentration sensor 70 is arranged within the wound dressing 20 (i.e., integrated in the wound dressing assembly 20) or at the connector 25 that fluidly connects the wound dressing 20 to the negative pressure source 14. In those instances where the gas concentration sensor 70 is provided externally to the housing 19, the gas concentration sensor 70 may comprise or otherwise be connected to a local communication module (e.g., a Bluetooth chip) in order to communicate with control circuitry 11.

Accordingly, the apparatus 10 is provided with a blockage-detection functionality in order to alert a user (i.e., a patient or a caregiver) of the apparatus 10 of the existence of a blockage condition in the wound treatment system 1, 1'. A blockage condition refers to an obstruction or restriction within the system 1, 1' that impedes the proper functioning of the therapy. This obstruction can occur anywhere along the NPWT system 1, 1', including the tubing 21, 41, canister 16, or the connectors. Blockages can result from factors such as kinking or twisting of tubing 21, 41, clotted blood or exudate within the tubing 21, 41, or occlusion of the wound dressing 20. Blockages prevent the effective removal of wound exudate and can compromise the therapeutic efficacy of NPWT, potentially leading to inadequate wound healing or other complications. Moreover, for single-lumen NPWT systems (e.g., the system 1 illustrated in Fig. 1), one can detect blockages between the gas concentration sensor 70 and the wound dressing 20 by checking the gas the concentration level of one or more gases (e.g., CO₂) after negative pressure has been established (e.g., at -135 mmHg or -125 mmHg) and the pump 14 has been turned off. Accordingly, the control circuitry 11 may be configured to output a blockage signal in response to the gas-concentration sensor being non-responsive while the source of negative pressure is inactive after negative pressure has been established at the wound site.

Further, the control circuitry 11 is configured to output a leakage signal indicative of a leakage being present within the fluid flow path, or within the system 1, 1' based on a detected concentration level of one or more gases within the fluid flow path over time. In particular, the control circuitry 11 may be configured to output the leakage signal indicative of a leakage being present within the fluid flow path based on a detected concentration level of one or more gases within the fluid flow path over time while the source of negative pressure 14 is inactive. In some embodiments, the control circuitry 11 may be configured to output the leakage signal indicative of a leakage being present within the fluid flow path in response to a detected concentration level of one or more gases decreasing while the source of negative pressure 14 is inactive. Here, the leakage signal trigger may be applied in response to the detected concentration level of one or more gases decreasing above a threshold so to reduce the risk of false positives (e.g., leakage alarm triggers due to small/negligible leakages). Alternatively, the control circuitry 11 may be configured to output the leakage signal indicative of a leakage being present within the fluid flow path in response to a detected concentration level of one or more gases decreasing more than a concentration level change rate value. In other words, one looks at how fast the concentration level is decreasing, and if it is decreasing faster than some set value, the leakage-detection functionality is triggered.

Accordingly, if the source of negative pressure 14 is inactive (i.e., turned off) the concentration level of the one or more gases (e.g., CO₂) is expected to be static (within some suitable margin of error). However, if it is nevertheless decreasing, and in particular if it is decreasing at a rapid pace, this is treated as an indicator of a leakage being present in the fluid flow path. In other words, the apparatus 10 is provided with a leakage-detection functionality in order to alert a user (i.e., a patient or a caregiver) of the apparatus 10 of the existence of a leakage condition in the wound treatment system 1, 1'. A leakage condition refers to the unintended loss of negative pressure from the wound dressing or the NPWT system. This loss of pressure can occur due to gaps or breaches in the sealing of the wound dressing 20, tubing 21, 41 connections, or the adhesive interface between the dressing 20 and the skin. Leakage compromises the effectiveness of NPWT by reducing the suction force applied to the wound, which can impair the removal of excess fluid, inhibit tissue contraction, and impede wound healing.

As mentioned, the apparatus 10 may comprise a user interface comprising one or more LEDs 62. Thus, in some embodiments, the control circuitry 11 may be configured to activate at least one LED of the one or more of the LEDs 62 in response to the blockage signal being output, and/or activate at least one LED of the one or more of the LEDs 62 in response to the leakage signal being output. In particular, the apparatus 10 may have a first LED serving as a blockage indicator and a second LED serving as a leakage indictor, whereupon the control circuitry 11 may be configured to activate the first LED in response to the blockage signal being output, and to activate the second LED in response to the leakage signal being output.

As mentioned, the apparatus may comprise a communication interface 56 interface having at least one antenna and at least one transceiver operatively connected to the at least one antenna. The control circuitry 11 may be is operatively connected to the communication interface and further configured to transmit a first signal to an external device in response to the blockage signal being output, and/or transmit a second signal to the external device in response to the leakage signal being output. The external device

The external device may be an external handheld device 50, such as a mobile phone (e.g. a smart phone) operatively connected to the apparatus 10. Moreover, the handheld device 50 preferably comprises corresponding hardware and software capabilities to establish a wireless communication link with the apparatus 10 and to transmit and receive signals to and from the apparatus 10. Moreover, the external device may comprise a suitable software application configured to output a Graphical User Interface (GUI) comprising a graphical representation comprising an indication of the blockage and/or the leakage in the wound treatment system 1 via a display apparatus of the external device 50. In more detail, depending on which signal (first or second) was received by the external device 50, the graphical representation may indicate whether there is blockage or a leakage detected in the wound treatment system 1, 1'.

Further, in some embodiments, the control circuitry 11 is configured to output a wound status signal indicative of a health status of the wound based on a detected concentration level of one or more gases within the fluid flow path over time. In particular, the gas concentration sensor may output a concentration level of Hydrogen Sulfide, Ammonia, Methane, or one or more VOCs - and the control circuitry 11 may be configured to determine a wound health status based on the concentration levels of one or more of these gases. Then, in response to the concentration level of one or more of these gases surpassing a set threshold, the control circuitry 11 may be configured to output a signal indicative of a potential infection being detected in the wound. For some or all of these gases, the threshold may be zero, in particular if they are not generally comprised in the atmospheric gases of the environment of the user. In more detail, bacteria and fungi can release various gases as metabolic byproducts, such as those mentioned above, and elevated concentration levels of one or more these gases could be used as an early indicator of a potential infection being present at the wound site. Thus, the gas concentration sensor 70 may be used to obtain early indications of infections being present at the wound site, and alert the caregiver/user so that proper remedies can be given at an early stage.

In some embodiments, the gas concentration sensor 70 is configured to detect a concentration level of water vapor (H₂O). Thereby, one can determine the humidity-level in the fluid flow path, and from that derive if the wound is a high-exuding wound (i.e., generating large amounts of exudate) or a low-exuding wound (i.e., generating small or no amounts of exudate). This may then be used to control the length of the pressure regulation cycles for dual-lumen NPWT systems 1', and in particular, the frequency of the flushing periods/sequences. In more detail, as mentioned in the foregoing, the primary purpose of the "flushing" is to introduce air into the system 1, 1' in order to be able to transport exudate away from the wound site 27. However, if one is given a real-time indication of the amount of exudate being generated by the wound, the one can effectively control the frequency of the flushing periods/sequences and reduce the energy consumption of the apparatus 10 in those situations when there is no exudate to be transported.

Thus, in some embodiments, the control circuitry 11 is configured to control a frequency, or timing of the flushing sequence(s) based on a detected concentration level of water vapor in the first fluid flow path or at the wound site. In other words, the control circuitry 11 may be configured to execute flushing sequences more or less often for a therapy cycle based on a detected concentration level of water vapor in the first fluid flow path or at the wound site. In particular, the control circuitry 11 may be configured to operate the apparatus 10 in a first mode in response to a detected concentration level of water vapor being above a first set value, and to operate the apparatus 10 in a second mode in response to the detected concentration level of water vapor being below a second set value. Here, the first and second set values may be the same or different values. The first mode may comprise shorter pressure regulation cycles while the second mode may comprise longer pressure regulation cycles. The set values may for example be dynamically configured by a user or caregiver during an initial period of using the system 1, 1' as each wound may be different to set some sort of baseline values of the concentration level of water vapor.

Moreover, in some embodiments, the control circuitry 11 may be configured to a frequency, or timing of the flushing sequence(s) based on a relative change of a detected concentration level of water vapor in the first fluid flow path or at the wound site. For example, should the output from the gas concentration sensor 70 indicate a decrease of the concentration level of water vapor in the first fluid flow path or at the wound site relative to an earlier period of time (e.g., 2 hours, 1 hour earlier, 30 minutes earlier, 20 minutes earlier, previous pressure regulation cycle(s), etc.), one can reduce the frequency of flushing sequence executions as compared to the earlier period of time so as to perform flushing more seldom. Similarly, should the output from the gas concentration sensor 70 indicate an increase of the concentration level of water vapor in the first fluid flow path or at the wound site relative to an earlier period of time (e.g., 2 hours, 1 hour earlier, 30 minutes earlier, 20 minutes earlier, previous pressure regulation cycle(s), etc.), one can increase the frequency of flushing sequence executions as compared to the earlier period of time so as to perform flushing more often.

A pressure regulation cycle may be understood as a time period comprising at least a "pressure regulation period" and a "flushing period". During the "pressure regulation period" the control circuitry 11 is configured to operate the negative pressure source 14 so to maintain a level of negative pressure in a suitable negative pressure range as exemplified above, i.e., the apparatus 10 is operated in a "pressure regulation mode". In other words, during the "pressure regulation period" the electronically controllable valve 40 is closed and the source of negative pressure 14 is operated to maintain a desired level of negative pressure at the wound site 27. During the "flushing period" the control circuitry 11 is configured to open the electronically controllable valve 40 so to introduce fluid (e.g., air from the ambient atmosphere) to the wound site 27, close the valve 40, and activate the source of negative pressure 14 to draw wound exudate from the wound site and re-establish the negative pressure level, i.e., the apparatus 10 is operated in a "flushing mode" or the apparatus 10 executes a "flushing sequence".

The durations of the pressure regulation periods and flushing periods, and accordingly the duration of a pressure regulation cycle, may be fixed (i.e., time-controlled) or dynamically controlled based pressure measurements. The duration of a pressure regulation cycle may also be dynamically controlled based on a total treatment time such that the pressure regulation cycles are shorter during an initial part of the treatment as compared to a later part. However, in some embodiments, the durations of the pressure regulation periods and flushing periods, and accordingly the duration of a pressure regulation cycle, may be controlled based on pressure measurements in combination with time-control. For example, during the flushing period the control circuitry 11 may be configured to open the valve until a pressure level P (as measured by the sensor(s) 15) is reached or for 2 seconds, whichever occurs first. Thus, the time-control aspect may define a longest duration for each period, while detection of certain pressure levels may shorten the duration. However, as mentioned, in some embodiments, the duration of the pressure regulation cycles is dynamically set based on a detected concentration level of water vapor in the fluid flow path (between the wound site 27 and the source of negative pressure 14).

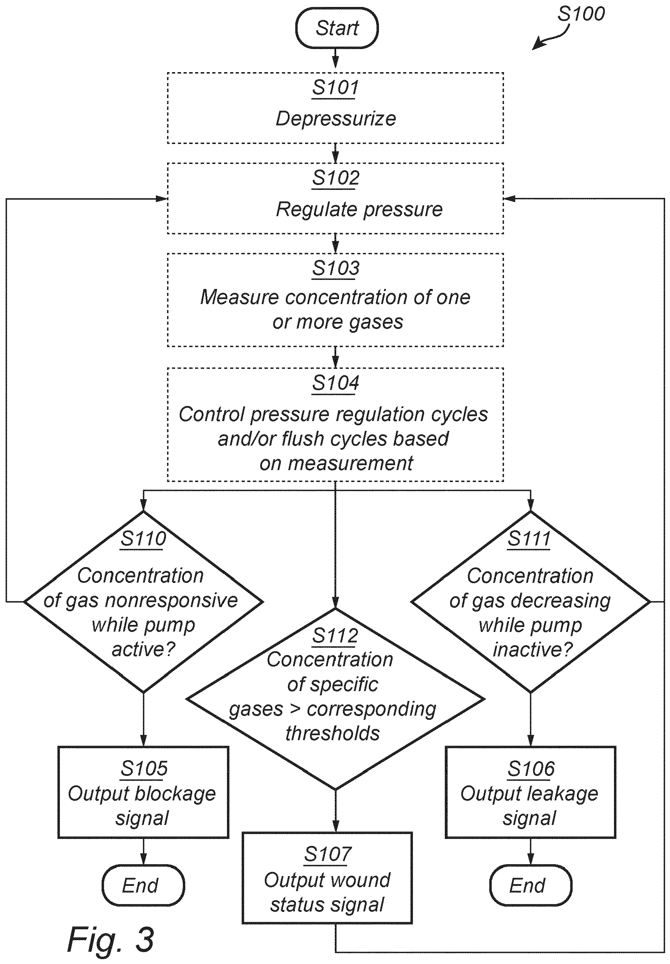

Fig. 3 is a schematic flowchart representation of a method for operating an apparatus for providing negative pressure to a wound site in accordance with some embodiments. The apparatus may be an apparatus according to any one of the embodiments disclosed herein. The apparatus may accordingly comprise a source of negative pressure configured to provide negative pressure via a fluid flow path from an inlet of the source of negative pressure to a wound site, and a gas concentration sensor configured to detect concentration levels of one or more gases within the fluid flow path or at the wound site. The method S100 is preferably a computer-implemented method S100, performed by a processing system of the apparatus. The processing system may for example comprise one or more processors and one or more memories coupled to the one or more processors, wherein the one or more memories store one or more programs that causes the apparatus to perform the steps, services and functions of any one of the embodiments of the method S100 disclosed herein when executed by the one or more processors.

In some embodiments, the method S100 comprises depressurizing S101 the wound site by activating the source of negative pressure. In other words, the method S100 may comprise turning the source of negative pressure on so to reduce the pressure level at the wound site via the first fluid flow path from an ambient pressure level. The source of negative pressure S101 may be activated S101 until a set pressure value (e.g., -125 mmHg) has been reached (as indicated by the one or more pressure sensors). The depressurization S101 is to be construed as a procedure that is generally only carried out upon initial start-up of the apparatus.

Further, the method S100 may comprise regulating S102 the pressure level at the wound site. In more detail, the method S100 may comprise maintaining S102, at the wound site, a pressure level that is between an upper pressure value and a lower pressure value, by activating and deactivating the negative pressure source. The upper pressure value and lower pressure value may for example be predefined or otherwise preconfigured in the apparatus. As mentioned, the upper pressure value may for example be -115 mmHg or -120 mmHg, while the lower pressure value may for example be -135 mmHg or -130 mmHg.

Further, the method S100 may comprise, measuring S103, using the gas concentration sensor, a concentration level of one or more gases within the fluid flow path. The method S100 may further comprise controlling S104 a duration of the pressure regulation cycles executed by the apparatus based on a detected/measured concentration level of water vapor in the fluid flow path.

The method S100 further comprises outputting S105 a blockage signal indicative of a blockage being present within the fluid flow path based on a detected S103 concentration level of one or more gases within the fluid flow path over time. In some embodiments, the method S100 comprises outputting S105 the blockage signal indicative of a blockage being present within the fluid flow path based on a detected S103 concentration level of one or more gases within the fluid flow path over time while the source of negative pressure is active. Accordingly, the method S100 may comprise outputting S105 the blockage signal indicative of a blockage being present within the fluid flow path in response to a detected concentration level of the one or more gases within the fluid flow path being non-responsive or non-changing S110 while the source of negative pressure is active.

The method S100 further comprises outputting S106 a leakage signal indicative of a leakage being present within the fluid flow path based on a detected S103 concentration level of one or more gases within the fluid flow path over time. In some embodiments, the method S100 comprises outputting S106 the leakage signal indicative of a leakage being present within the fluid flow path based on a detected S103 concentration level of one or more gases within the fluid flow path over time while the source of negative pressure is inactive. In some embodiments, the method S100 may comprise outputting S106 the leakage signal indicative of a leakage being present within the fluid flow path in response to a detected concentration level of one or more gases decreasing S111 while the source of negative pressure is inactive. Here, the leakage signal trigger may be applied in response to the detected concentration level of one or more gases decreasing above a threshold so to reduce the risk of false positives (e.g., leakage alarm triggers due to small/negligible leakages). Alternatively, the method S100 may comprise outputting S105 the leakage signal indicative of a leakage being present within the fluid flow path in response to a detected concentration level of one or more gases decreasing Sill more than a concentration level change rate value. In other words, one looks at how fast the concentration level is decreasing, and if it is decreasing faster than some set value, the leakage-detection functionality is triggered.

Furthermore, the method S100 may comprise activating at least one LED of the one or more of the LEDs in response to the blockage signal being output, and/or activating at least one LED of the one or more of the LEDs in response to the leakage signal being output. In particular, the apparatus may have a first LED serving as a blockage indicator and a second LED serving as a leakage indictor, whereupon the first LED may be activated in response to the blockage signal being output, and the second LED may be activated in response to the leakage signal being output.

Still further, the method S100 may comprise transmitting, using a communication interface, a first signal to an external device in response to the blockage signal being output, and/or transmitting, via the communication interface, a second signal to the external device in response to the leakage signal being output.

The method S100 may further comprise outputting S107 a wound status signal indicative of a health status of the wound based on a detected concentration level of one or more gases within the fluid flow path over time. As mentioned, the gas concentration sensor may output a concentration level of Hydrogen Sulfide, Ammonia, Methane, or one or more VOCs - and the method S100 may comprise determining a wound health status based on the concentration levels of one or more of these gases.

Then, in response to the concentration level of one or more of these gases surpassing S112 a set threshold, the method S100 may comprise outputting S107 a signal indicative of a potential infection being detected in the wound. For some or all of these gases, the threshold may be zero, in particular if they are not generally comprised in the atmospheric gases of the environment of the user.

Executable instructions for performing these functions are, optionally, included in a non-transitory computer-readable storage medium or other computer program product configured for execution by one or more processors of an apparatus for providing reduced pressure to a wound dressing.

The herein disclosed technology has been presented above with reference to specific embodiments. However, other embodiments than the above described are possible and within the scope of the claims. Different method steps than those described above, performing the method by hardware or software, may be provided within the scope of the claims. Thus, according to some embodiments, there is provided a non-transitory computer-readable storage medium storing one or more programs configured to be executed by one or more processors of an apparatus of a reduced-pressure wound treatment system, the one or more programs comprising instructions for performing the method S100 according to any one of the above-discussed embodiments. According to some embodiments, there is provided a computer program product comprising instructions which, when the program is executed by a computing device of an apparatus for providing negative pressure to a wound site, causes the apparatus to carry out the method S100 according to any one of above-discussed embodiments.

Generally speaking, a computer-accessible medium may include any tangible or non-transitory storage media or memory media such as electronic, magnetic, or optical media coupled to computer system via bus. The terms "tangible" and "non-transitory," as used herein, are intended to describe a computer-readable storage medium (or "memory") excluding propagating electromagnetic signals, but are not intended to otherwise limit the type of physical computer-readable storage device that is encompassed by the phrase computer-readable medium or memory. For instance, the terms "non-transitory computer-readable medium" or "tangible memory" are intended to encompass types of storage devices that do not necessarily store information permanently, including for example, random access memory (RAM). Program instructions and data stored on a tangible computer-accessible storage medium in non-transitory form may further be transmitted by transmission media or signals such as electrical, electromagnetic, or digital signals, which may be conveyed via a communication medium such as a network and/or a wireless link.

The processor(s) 11 (associated with the apparatus 10) may be or include any number of hardware components for conducting data or signal processing or for executing computer code stored in memory. The device 10 may accordingly have an associated memory, and the memory may be one or more devices for storing data and/or computer code for completing or facilitating the various methods described in the present description. The memory may include volatile memory or non-volatile memory. The memory may include database components, object code components, script components, or any other type of information structure for supporting the various activities of the present description. According to some embodiments, any distributed or local memory device may be utilized with the systems and methods of this description. According to some embodiments embodiment the memory is communicably connected to the processor 11 (e.g., via a circuit or any other wired, wireless, or network connection) and includes computer code for executing one or more processes described herein.

It should be noted that any reference signs do not limit the scope of the claims, that some embodiments may be at least in part implemented by means of both hardware and software, and that several "means" or "units" may be represented by the same item of hardware.

Although the figures may show a specific order of method steps, the order of the steps may differ from what is depicted. In addition, two or more steps may be performed concurrently or with partial concurrence. Such variation will depend on the software and hardware systems chosen and on designer choice. All such variations are within the scope of the appended claims. Likewise, software implementations could be accomplished with standard programming techniques with rule-based logic and other logic to accomplish the generating steps, activating steps, operating steps, causing steps, steps. The above mentioned and described embodiments are only given as examples and should not be limiting to the appended claims. Other solutions, uses, objectives, and functions within the scope of the below described patent claims should be apparent for the person skilled in the art.

## Claims

1. An apparatus (10) for providing negative pressure to a wound site (27), the apparatus (10) comprising:
a source of negative pressure (14) configured to provide negative pressure via a fluid flow path from an inlet of the source of negative pressure to a wound site;
a gas concentration sensor (70) configured to detect concentration levels of one or more gases within the fluid flow path or at the wound site;
control circuitry (11) operatively connected to the source of negative pressure (14) and the gas concentration sensor (70), wherein the control circuitry is configured to:
output a blockage signal indicative of a blockage being present within the fluid flow path based on a detected concentration level of one or more gases within the fluid flow path over time; and/or
output a leakage signal indicative of a leakage being present within the fluid flow path based on a detected concentration level of one or more gases within the fluid flow path over time.

2. The apparatus (10) according to claim 1, wherein the control circuitry (11) is configured to:
output a wound status signal indicative of a health status of the wound based on a detected concentration level of one or more gases within the fluid flow path over time.

3. The apparatus (10) according to claim 1 or 2, wherein the control circuitry (11) is configured to:
output the blockage signal indicative of a blockage being present within the fluid flow path based on a detected concentration level of one or more gases within the fluid flow path over time while the source of negative pressure (14) is active.

4. The apparatus (10) according to any one of claims 1-3, wherein the control circuitry (11) is configured to:
output the leakage signal indicative of a leakage being present within the fluid flow path based on a detected concentration level of one or more gases within the fluid flow path over time while the source of negative pressure (14) is inactive.

5. The apparatus (10) according to any one of claims 1-4, further comprising a user-interface comprising one or more Light Emitting Diode, LEDs (62);
wherein the control circuitry (11) is operatively connected to the user interface and further configured to:
activate at least one LED of the one or more of the LEDs (62) in response to the blockage signal being output, and/or
activate at least one LED of the one or more of the LEDs (52) in response to the leakage signal being output.

6. The apparatus (10) according to any one of claims 1-5, further comprising a communication interface (59) having at least one antenna and at least one transceiver operatively connected to the at least one antenna; and
wherein the control circuitry (11) is operatively connected to the communication interface (56) and further configured to:
transmit a first signal to an external device (50) in response to the blockage signal being output, and/or
transmit a second signal to the external device (50) in response to the leakage signal being output.

7. A computer-implemented method (S100) for operating an apparatus (10) for providing negative pressure to a wound site, wherein the apparatus comprises a source of negative pressure configured to provide negative pressure via a fluid flow path from an inlet of the source of negative pressure to a wound site, and a gas concentration sensor configured to detect concentration levels of one or more gases within the fluid flow path or at the wound site, the method comprising:
outputting (S105) a blockage signal indicative of a blockage being present within the fluid flow path based on a detected concentration level of one or more gases within the fluid flow path over time; and/or
outputting (S106) a leakage signal indicative of a leakage being present within the fluid flow path based on a detected concentration level of one or more gases within the fluid flow path over time.

8. The method (S100) according to claim 7, further comprising:
outputting (S107) a wound status signal indicative of a health status of the wound based on a detected concentration level of one or more gases within the fluid flow path over time.

9. The method (S100) according to claim 7 or 8, further comprising:
outputting (S105) the blockage signal indicative of a blockage being present within the fluid flow path based on a detected concentration level of one or more gases within the fluid flow path over time while the source of negative pressure is active.

10. The method (S100) according to any one of claims 7-9, further comprising:
outputting (S106) the leakage signal indicative of a leakage being present within the fluid flow path based on a detected concentration level of one or more gases within the fluid flow path over time while the source of negative pressure is inactive.

11. The method (S100) according to any one of claims 7-10, wherein the apparatus further comprises a user-interface comprising one or more Light Emitting Diode, LEDs, the method further comprising:
activating at least one LED of the one or more of the LEDs in response to the blockage signal being output (S105), and/or
activating at least one LED of the one or more of the LEDs in response to the leakage signal being output (S106).

12. The method (S100) according to any one of claims 7-11, wherein the apparatus further comprises a communication interface having at least one antenna and at least one transceiver operatively connected to the at least one antenna, the method further comprising:
transmitting a first signal to an external device in response to the blockage signal being output (S105), and/or
transmitting a second signal to the external device in response to the leakage signal being output (S106).

13. A computer program product comprising instructions which, when the program is executed by a computing device of an apparatus for providing negative pressure to a wound site, causes the apparatus to carry out the method (S100) according to any one of claims 7-12.

14. A system (1, 1') comprising:
an apparatus (10) according to any one of claims 1-6;
a wound cover (22) for creating a sealed space defined in part by the wound site (27); and
a tubing assembly defining the fluid flow path.
